# EUROPEAN PATENT SPECIFICATION

(11) **EP 1 129 211 B1**
(45) Date of publication and mention of the grant of the patent: **09.07.2003**
(21) Application number: 99954225.1
(22) Date of filing: 11.11.1999
(51) Int. Cl.: C12Q 1/00, G01N 35/00, G01N 27/30, G01N 27/327

(54) **ELECTRODE STRIPS FOR TESTING SMALL VOLUMES**
ELEKTRODENSTREIFEN ZUM TESTEN VON KLEINEN VOLUMEN
BANDES ELECTRODES PERMETTANT DE TESTER DES PETITS VOLUMES

(30) Priority: 11.11.1998 GB 9824627
(43) Date of publication of application: 05.09.2001
(73) Proprietor: Cambridge Sensors Ltd., Godmanchester, Cambs PE29 2XG (GB)
(72) Inventor: YON HIN, Bernadette Cambridge Sensors Limited, Cambridge CB4 1XT (GB); MCCANN, James Cambridge Sensors Limited, Cambridge CB4 1XT (GB); BLAIR, Neil, Hardwick Cambridge CB3 7XU (GB); COX, Lorna, Jean Cambridge Sensors Limited, Cambridge CB4 1XT (GB)
(74) Representative: Perry, Robert Edward
(86) International application number: GB9903764
(87) International publication number: WO00028068

(56) References cited:
- EP-A- 0 230 472
- EP-A- 0 593 096
- WO-A-97/02487
- WO-A-99/13100
- US-A- 4 218 421
- US-A- 5 169 600
- US-A- 5 628 890
- US-A- 5 679 311
- US-A- 5 779 867
- US-A- 5 798 031

## Description

### Field of the Invention

This invention relates to electrode strips for testing small volumes of, say, whole blood.

### Background of the Invention

Diabetes is one of the most common endocrine conditions. Sufferers must monitor their blood glucose level frequently. This is usually achieved by the use of small test strips which detect blood glucose.

Problems commonly experienced by users of these test strips are an inadequate amount of blood on the test strip and bad placement of the blood on the test strip. A number of devices have addressed this problem by using sample chambers that fill by capillary action. The sample is retained in close proximity to the electrodes which facilitate the measurement of the specific analyte in the sample; see EP-A-0170375 and US-A-5141868.

Such known devices comprise electrodes deposited on a non-conducting substrate, coated with a reagent system specific for the analyte of interest and housed within a cavity whose dimensions are sufficiently small to allow introduction of a sample, e.g. 2.5-3 µL in volume, by capillary action. The extent to which these devices can be miniaturized is limited by both the manufacturing tolerances and the signal-to-noise ratio achievable with their chemistry.

US-5820551 discloses a test strip comprising a support carrying a working electrode and a counter electrode, and an enzyme and a mediator that are coated on the active electrode. A drop of whole blood can provide a conducting path between the electrodes, and the concentration of glucose in the blood can be determined. The active electrode is exposed to a whole blood sample without an intervening membrane or other whole blood filter.

WO-A-98/55856 (published after the priority date claimed for this Application) discloses an analyte-specific reagent coated on the conductive layer, and a monofilament mesh laid over the reagent and the reference electrode. A sample application area is provided at one edge of the mesh.

### Summary of the Invention

According to the present invention, a test strip comprises a support carrying an active electrode and a counterelectrode, and a monofilament mesh or membrane within which a small volume of liquid to be tested can be distributed and provide contact between the electrodes, and wherein an analyte-specific reagent such as one component of a redox reaction, e.g. an enzyme, co-factor or mediator, is coated on the mesh or membrane. The test strip also includes a sample application area at one edge of the mesh or membrane. In particular, the invention provides a test strip for blood glucose, in which the sample requirement is very small, and efficient reaction kinetics are achieved by the application of the reagents in a novel manner.

### Description of the Invention

In accordance with this invention, any one or more of the components of a redox reaction, e.g. an enzyme such as glucose oxidase or glucose dehydrogenase, a co-factor and a mediator may be applied to a mesh or membrane which is placed over the device. For the purpose of illustration only, the invention may be described with reference to an enzyme-coated mesh. Whichever component or components are used, when the sample is added, they are solubilised quickly and form an efficient reaction medium that can provide contact between the separate electrodes of the test strip. In this manner, the reaction will proceed rapidly and without diffusion barriers. This reaction configuration is particularly indicated in cases where the sample volume is low, the sample is viscous (such as with whole blood) and a rapid reaction is required.

In a typical embodiment of the invention, the sensor test strip consists of two electrodes, one of which acts as a working electrode and another which acts as a counter, reference electrode. The end of the working electrode that is exposed to the sample has a mediator in intimate contact with it. The test strip effectively provides a reaction chamber defined by these two electrodes and an additional sheet, overlying the electrodes, which has been pre-coated with the redox enzyme and any necessary co-factor for that enzyme. The reaction chamber may also comprise further sheets of material and/or wetting agents, e.g. a surfactant, or cell-lysing materials (which may be placed in any one of the overlying sheets). In this manner, the active enzyme is not coated onto the conductor which forms the working electrode but is provided in a separate layer above it which, in tum, effectively forms the solution phase of the reaction chamber. When combined with lateral flow, conditions are created that approach efficient mixing in a stirred reaction chamber.

In an example of the invention, a silver chloride/silver reference/counter electrode is located adjacent to a carbon electrode. Typically, for this purpose, a pair of printed carbon electrodes is printed on a non-conducting substrate, and then silver/silver chloride is printed on one of the carbon electrodes to function as the reference/counter electrode. A non-conducting ink is printed over the carbon electrodes and the substrate, in order to define a portion of each electrode as a contact pad for insertion into a meter and another portion on each electrode away from the contact pad as the sensing area where the sample is received.

A mediator for the enzyme cofactor NADH is then prepared and deposited onto the electrode from aqueous solution by pipetting. Afurther layer containing NAD is then deposited onto the working electrode.

A monofilament mesh material is coated with a surfactant and then with a solution containing glucose dehydrogenase via pipetting, ink jet-coating or dip-coating, and is placed over the two electrodes to form a reaction chamber. This reaction chamber may be defined further by additional printing, or by the use of a top layer to form an edge fill cavity. For example, a second non-conducting ink printed on top of the mesh material, and then a cover tape is applied on top of the mesh in such a way as to leave an extended area of the mesh exposed for sample application.

The device allows the application of a small volume of sample (typically 1 µL or less) to the mesh extension. This is followed by flooding of the device sensing area with sample, bringing it into intimate contact with the measuring electrodes.

Devices having an edge fill are described in WO-A-98/55856. They can be simply adapted, in accordance with the present invention. In particular, reference may be made to Fig. 1 in WO-A-98/55856; components of this invention are the support (1), electrodes (2/3), mesh material (6) and tape (7); in addition, reagent is provided on the mesh material. Such a device can work by application at its edge, to a sample. This is particularly valuable in cases where it is difficult to extract the sample. Other configurations will be evident to one skilled in the art, including combinations of one or more of the cofactor, mediator or the enzyme coated onto the overlying mesh or membrane sheets. The choice of combination may on the reaction kinetics of the various compounds.

In another embodiment of the device, the enzyme or the mediator is coated on the sheet, the co-factor and the other of the mediator or the enzyme are coated onto the working electrode directly, and the sheet is capable of filtering the whole blood such that the active electrode sees a sample which is effectively free of whole blood cells. In this case, the haematocrit dependency of the result is substantially reduced. In this manner, the cell-filtering function of a selected membrane may be combined with the rapid kinetics of having the some or all of the active elements of the reaction (the enzyme, mediator and the co-factor) in the membrane, to produce a highly effective device.

In summary, according to the present invention, a device is constructed by depositing one or more of the reagents required for the quantitation of an analyte as a single or multiple layers on a fine mesh material or membrane; the deposited areas are of dimensions small enough to wet with a very small sample volume. The mesh or membrane can be used in both colorimetric and electrochemical devices.

A characteristic of this invention is that a reagent is applied precisely onto a target area on a woven material such as polyester or nylon or other porous membrane. In use, this provides rapid solubilisation of the reagents in the presence of the sample. The reagent or reagents can be applied in a number of different methods that result in the deposition of a known volume at a precise location and in a well-defined foot-print. These include the use of dispensing equipment such as a piston pump, syringe pump or on-demand ink-jet printer.

Also described is a flexible tape containing one or more reagents may be laminated to another flexible tape on which is printed a series of electrodes. Instead of cutting out individual sensors, the laminate (comprising a row or series of sensors) may be used sequentially, e.g. on being dispensed from a suitable dispenser. For this purpose, whether or not as a laminate, a tape of the invention may be provided as a roll, and stored in sealed cassettes which may also contain desiccant. In use, the cassette may be inserted into a automatic dispenser from which the tape is wound out automatically by an indexing mechanism to reveal sequentially the discrete sensors. The action of this instrument is therefore analogous to the action of a film in a camera. In this embodiment, the tape may also contain a red blood cell-lysing reagent such as saponin, in order to reduce the effect of haematocrit and haemoglobin in a whole blood sample. The tape may be further protected from moisture by being covered with a peelable film (e.g. of aluminium) that is automatically peeled off when the tape is dispensed from the cassette. When the sample is applied to the sensor, the amount of analyte of interest in the sample may be determined electrochemically. Such determination can be conducted by known methods.

The following Example illustrates the invention.

### Example

A conductive ink material is printed onto a non-conducting polyester sheet material by a screen-printing process. The conductive ink material consists of a mixture of graphite and carbon particles and a polymer binder in an organic solvent. After deposition of the conductive ink, solvents are removed in a forced air oven. A silver/silver chloride reference/counter electrode is printed onto one of each pair of printed carbon electrodes followed by a non-conducting ink layer to define the contact pads and the sensor area.

A mediator such as Meldola Blue, Nile Blue or other suitable dye and the enzyme co-factor nicotinamide adenine dinucleotide (NAD) are deposited onto the carbon electrode. Alternatively, the NAD is applied separately over the mediator from an aqueous ink.

The enzyme glucose dehydrogenase is deposited as uniform spots on a monofilament polyester mesh tape. This is achieved as follows:
(a) in a contact mode, where a drop formed at a dispenser tip in close proximity to the mesh is allowed to be transferred to the mesh by touching off the drop onto the mesh surface; or
(b) in a non-contact mode, where a drop formed by an ink-jet printhead or other orifice above the mesh is dropped onto the mesh from a distance under conditions which do not cause it to penetrate the mesh.

Upon drying, the spots spread to cover an area defined partly by the characteristics of the mesh weave and partly by the application conditions. Typically the areas covered by a 500 nL drop is 1.3 x 1.2 mm. The mesh tape is allowed to dry at room temperature.

The enzyme-modified mesh tape is then laminated onto the modified sheet of devices and secured further by a non-conducting print. Finally, a cover tape is laminated on tope of the mesh. The sheets of devices are disc cut into individual devices. In an alternative device format, the laminated sheets are wound and included in a cassette type unit, allowing a single device to be used by a wind-on mechanism similar to a camera film-winding system.

## Claims

1. A test strip comprising a support carrying an active electrode and a counterelectrode, and a monofilament mesh or membrane within which a small volume of liquid to be tested can be distributed and provide contact between the electrodes, wherein an analyte-specific reagent is coated on the mesh or membrane, and wherein the test strip includes a sample application area at one edge of the mesh or membrane.

2. A test strip according to claim 1, wherein the reagent is at least one component of a redox reaction.

3. A test strip according to claim 2, wherein the at least one compound is one or more of an enzyme, a mediator and/or co-factor for the enzyme.

4. A test strip according to claim 2, wherein the at least one component comprises an enzyme.

5. A test strip according to claim 3 or claim 4, wherein the enzyme is glucose oxidase or glucose dehydrogenase.

6. A method for testing a liquid for the presence of an analyte, which comprises contacting the liquid with a test strip according to any of claims 1 to 5, and detecting the current.

7. A method according to claim 6, wherein the liquid is blood and the analyte is glucose.

## Patentansprüche

1. Teststreifen, umfassend einen eine aktive Elektrode und eine Gegenelektrode tragenden Träger sowie ein Monofilament-Gewebe bzw. eine Membran, worin ein kleines Volumen einer zu testenden Flüssigkeit verteilt werden und als Kontakt zwischen den Elektroden dienen kann, wobei das Gewebe bzw. die Membran mit einem analytspezifischen Reagens beschichtet wird und wobei der Teststreifen einen Probenauftragsbereich an einem Ende des Gewebes bzw. der Membran enthält.

2. Teststreifen nach Anspruch 1, wobei es sich bei dem Reagens um wenigstens eine Komponente einer Redoxreaktion handelt.

3. Teststreifen nach Anspruch 2, wobei es sich bei der wenigstens einen Verbindung um ein oder mehrere Enzyme, einen Mediator und/oder einen Cofaktor für das Enzym handelt.

4. Teststreifen nach Anspruch 2, wobei die wenigstens eine Komponente ein Enzym umfaßt.

5. Teststreifen nach Anspruch 3 oder Anspruch 4, wobei es sich bei dem Enzym um Glucose-Oxidase oder Glucose-Dehydrogenase handelt.

6. Verfahren zum Testen einer Flüssigkeit auf die Anwesenheit eines Analyten, wobei die Flüssigkeit mit einem Teststreifen nach einem der Ansprüche 1 bis 5 in Kontakt gebracht und der Stromfluss nachgewiesen wird.

7. Verfahren nach Anspruch 6, wobei es sich bei der Flüssigkeit um Blut und bei dem Analyten um Glucose handelt.

## Revendications

1. Bande d'essai comprenant un support portant une électrode active et une contre-électrode, et un filet monofilamentaire ou membrane à l'intérieur duquel un faible volume de liquide à tester peut être réparti et assurer un contact entre les électrodes, dans laquelle un réactif particulier à un analyte est appliqué sur le filet ou la membrane et dans laquelle la bande de test comprend une zone d'application d'échantillon en un bord du filet ou de la membrane.

2. Bande d'essai selon la revendication 1, dans laquelle le réactif est au moins un composant d'une réaction d'oxydoréduction.

3. Bande d'essai selon la revendication 2, dans laquelle le au moins un composant est l'un ou plusieurs d'une enzyme, d'un médiateur et/ou un cofacteur pour l'enzyme.

4. Bande d'essai selon la revendication 2, dans laquelle le au moins un composant comprend une enzyme.

5. Bande d'essai selon la revendication 3 ou la revendication 4, dans laquelle l'enzyme est une oxydase de glucose ou une déshydrogénase de glucose.

6. Procédé de test d'un liquide en présence d'un analyte, qui comprend la mise en contact du liquide avec une bande d'essai selon l'une quelconque des revendications 1 à 5, et à détecter le courant.

7. Procédé selon la revendication 6, dans lequel le liquide est le sang et l'analyte est le glucose.
